# EUROPEAN PATENT SPECIFICATION

(11) **EP 1 515 776 B1**
(45) Date of publication and mention of the grant of the patent: **16.04.2008**
(21) Application number: 03705594.4
(22) Date of filing: 14.02.2003
(51) Int. Cl.: A61N 1/368, A61N 1/37, A61B 5/0452

(54) **A HEART MONITORING AND STIMULATING DEVICE, A SYSTEM INCLUDING SUCH A DEVICE**
VORRICHTUNG ZUR ÜBERWACHUNG UND STIMULIERUNG DES HERZENS; EINE SOLCHE VORRICHTUNG ENTHALTENDES SYSTEM
DISPOSITIF DE STIMULATION ET DE SURVEILLANCE CARDIAQUES, SYSTEME COMPRENANT UN TEL DISPOSITIF

(30) Priority: 14.06.2002 SE 0201822
(43) Date of publication of application: 23.03.2005
(73) Proprietor: St Jude Medical AB, 175 84 Järfälla (SE)
(72) Inventor: BJÖRLING, Anders, S-175 53 Järfälla (SE); HOLMSTRÖM, Nils, S-175 57 Järfälla (SE)
(74) Representative: Kalling, Sven Owe
(86) International application number: PCT/SE2003/000245
(87) International publication number: WO 2003/105952

(56) References cited:
- US-A- 5 626 620
- US-A- 5 720 768

## Description

### BACKGROUND OF THE INVENTION

### 1. Field of the invention

The present invention relates to an implantable heart monitoring and stimulating device comprising a control circuit and a memory connected to said control circuit. The control circuit is adapted to be connected to at least a first and a second sensing member. The first sensing member is adapted for sensing cardiac events related to the right ventricle of the heart and the second sensing member is adapted for sensing cardiac events related to the left ventricle of the heart. The control circuit is arranged to be able to receive signals from said first and second sensing members such that cardiac events of said right and left ventricles are detectable by the control circuit. The control circuit is also adapted to be connected to at least a first and a second stimulation member. The first stimulation member is adapted for electrically stimulating the right ventricle of the heart and the second stimulation member is adapted for electrically stimulating the left ventricle of the heart. The control circuit is arranged to be able to transmit stimulation pulses to said first and second stimulation members in order to stimulate said right and left ventricles.

The invention also relates to a system including such a device and to the use of the system. The device may be used to monitor the performance of a heart of a human or animal being as well as to stimulate said heart.

### 2. Description of the prior art

A device of the above described kind is known in the art. Several different implantable devices for monitoring and stimulating a heart are known. The devices are normally able to sense the electrical activity of the heart. It is also known to sense other physiological parameters, such as blood pressure, oxygen level etc. Some devices are also able to sense the activity level of the human or animal being into which the device is implanted. With such a device it is thus for example possible to detect whether the person or animal in question is exercising or resting. Some implantable devices are able to deliver stimulation pulses to both the left and right ventricles of the heart, and sometimes also to the left and right atria.

US-A-5 720 768 describes different possible electrode positions in order to stimulate or sense the different chambers of the heart. This document discloses the features of the preamble of claim 1.

US-A-6 070 100 describes that electrodes may be positioned in both the left and the right atrium as well as in the left and the right ventricles.

US-A-5 213 098 describes a cardiac stimulator with an oxygen saturation sensor positioned in the coronary sinus of the heart. This device is also able to sense the blood pressure and the electrical activity of the heart.

US-A-5 199 428 describes a device for detecting myocardial ischemia. A pH sensor or an oxygen saturation sensor may be positioned in the coronary sinus.

US-A-6 236 873 B1 describes an electrochemical sensor for measuring the oxygen content in blood.

US-A-4 202 339 describes a sensor for measuring the oxygen saturation level in blood.

US-A-4 453 537 describes a device for sensing, inter alia, the carbon dioxide content in the blood.

US-A-5 582 170 describes a fibre optic sensor for sensing the nitric oxide content in blood.

It is also known to communicate with an implanted heart monitor-ing/stimulating device in a wireless manner, i.e. with the help of so-called telemetry. This can be done by inductive communication or via radio waves. With the help of telemetry it is thus for example possible to obtain information about the status of an implanted device. It is also known to input new information into the device with the help of such telemetry.

### SUMMARY OF THE INVENTION

An object of the invention is to provide an implantable heart monitoring and stimulating device of the kind described in the first paragraph above and in which the change in the heart condition can be monitored in a suitable manner. A further object is to provide such a device in which the condition of the heart of a patient who suffers from cardiomyopathy can be monitored. A still further object is to provide such a device in which the condition of the heart of a patient suffering from a bundle branch block can be monitored in an efficient manner. Still another object is to provide such a device in which the monitored result concerning the heart condition is easy to understand for, for example, a physician.

The above objects are achieved by an implantable heart monitoring device of the kind described in the first paragraph above, but wherein said control circuit also is arranged to at least enable the following:
a) determine the time, within a heart cycle, between a first event related to the right ventricle and a second event related to the left ventricle, or vice versa,
b) store the determined time in said memory,
wherein said control circuit is arranged to perform steps a) and b) at a plurality of occasions and to store the corresponding determined times in said memory.

By determining said times and storing the times in the memory, the memory contains information concerning the heart condition at the different occasions. This information may then be used to analyse how the heart condition has changed with time. The invention is for example useful in connection with a patient suffering from a dilated cardiomyopathy. In particular, a left bundle branch block can in many cases lead to a dilated cardiomyopathy. Thereby the patient's left ventricle will be enlarged. This will affect the cardiac performance of the patient. The enlargement of the left ventricle may lead to a slower contraction of the left ventricle. This makes the burden on the left ventricle higher, which may lead to a further enlargement of this ventricle. The underlying cause of this condition, for example caused by a left bundle branch block, is that the left and right ventricles are not synchronised with each other. The synchronisation may be improved by using an implanted biventricular pacemaker. When the heart condition is improved, such that the left and right ventricles will be more synchronised, the size of the left ventricle is likely to decrease. This may improve the conduction properties of the heart. The delay between events of the right and left ventricles will therefore become smaller. With the present invention it is therefore possible to analyse whether the heart condition improves or gets worse with regard to the mention condition.

It may be noted that at each occasion, the mentioned time may be determined once and stored. It is also possible that the mentioned time is determined several times at the mentioned occasion and that a relationship between the different determined times (for example an average value of the determined times) at the occasion is stored in the memory.

According to one preferred embodiment of the invention, the mentioned first event is a sensed R-wave of one ventricle and the second event is a corresponding sensed R-wave in the other ventricle of the heart. The control circuit is arranged such that no stimulation pulses are delivered to the stimulation members at least for the heart cycles when said time is determined between the sensed R-waves.

According to another embodiment, the control circuit is arranged such that the first event is the delivery of a stimulation pulse to one ventricle and such that the second event is a sensed R-wave in the other ventricle. The control circuit is hereby arranged such that no stimulation pulse is delivered to the ventricle in which the R-wave is sensed, at least for the heart cycle when the time is determined.

It should be noted that the above described manners of determining the time may also be combined such that both the time between two R-waves and the time between a stimulation pulse and the R-wave are determined.

All these determined times relate to how much delay there is between events in the left and the right ventricles. Therefore, by monitoring how these times differ between the different occasions, the change of the heart condition may be monitored.

According to a further embodiment, the control circuit is arranged also to sense the activity level of a human or animal being in which the device is implanted. The control circuit is hereby arranged to choose said occasions to carry out the determination of the time when the activity level is of a predetermined level or within a predetermined range. Preferably, the activity level during said occasions implies a low level of activity. The determination of the time may for example be carried out when the human or animal being is asleep. By always determining said times at approximately the same level of activity, an accurate indication of the change of the heart condition is obtained.

The control circuit may for example be arranged such that the time or times are determined at least once a month, preferably at least once a week. The control circuit is preferably arranged such that the time or times are not determined too often, for example not more often than each hour or preferably not more often than once a day. With such intervals between said occasions, a sufficient indication of the change of the heart condition is obtained without using too much space is said memory. It should however be noted that it is of course possible that the time or times are determined more often than once each hour.

According to another preferred embodiment, the device and the control circuit can be arranged to detect at least one pacing or physiological parameter such as the pacing rate, blood pressure, oxygen content in the blood, metabolic substance in the blood or the activity level of the human or animal being in which the device is implanted, wherein the control circuit is arranged to determine said times at different values of said parameter. The times may thus be determined for example at a higher pacing rate and at a lower pacing rate. The different determined values may be useful in further analysing the heart condition. Similar determination may be done for other pacing or physiological parameters.

According to another aspect of the invention, the invention provides an implantable heart monitoring and stimulating system comprising a device according to any of the above embodiments and a first and a second lead connected to the device. The first sensing member is thereby arranged on the first lead and the second sensing member is arranged on the second lead. Preferably, also the first stimulation member is arranged on the first lead and the second stimulation member is arranged on the second lead. According to one preferred embodiment, the same member may be used for the first sensing member and the first stimulation member. Similarly, the same member may be used for the second stimulation member and the second sensing member. Such a device may thus be implanted in a human or animal being. With such a device the above mentioned advantages are achieved.

There is also disclosed the use of such a system. According to this use, the system is implanted in a human or animal being and the first sensing member is positioned in or at the right ventricle of the heart of said human or animal being wherein the second sensing member is positioned in or at the left ventricle of said heart. This use thus provides an advantageous manner of using the system.

According to a preferred manner of using the system, the values of the determined times stored in the memory are transferred to an external apparatus which does not form part of the implanted system. For example, the determined values may be transferred by telemetry, such as is known to a person skilled in the art. With the help of the external apparatus, it is thereby possible to analyse the stored values in order to find out how the heart condition has changed with time.

According to a preferred use, a graph which represents the change of the heart condition of said human or animal being is produced. The system is particularly useful in order to monitor the change of the heart condition for a human or animal being suffering from cardiomyopathy, for example caused by a bundle branch block.

### BRIEF DESCRIPTION OF THE DRAWINGS

- Fig 1: shows schematically a heart monitoring system with a heart monitoring device connected to leads with sensing and stimulation members positioned in a heart.
- Fig 2: shows schematically on a time scale sensed R-waves in the two ventricles of a heart.
- Fig 3: shows schematically on a time scale a delivered stimulation pulse to the right ventricle and a sensed R-wave of the left ventricle.
- Fig 4: shows schematically on a time scale a stimulation pulse delivered to the left ventricle and a sensed R-wave of the right ventricle.
- Fig 5: shows schematically a flow chart of the function of a heart monitoring system according to an embodiment of the invention as well as the use of such a system.
- Fig 6: shows schematically how a time determined at different occasions may change with time.

### DESCRIPTION OF PREFERRED EMBODIMENTS

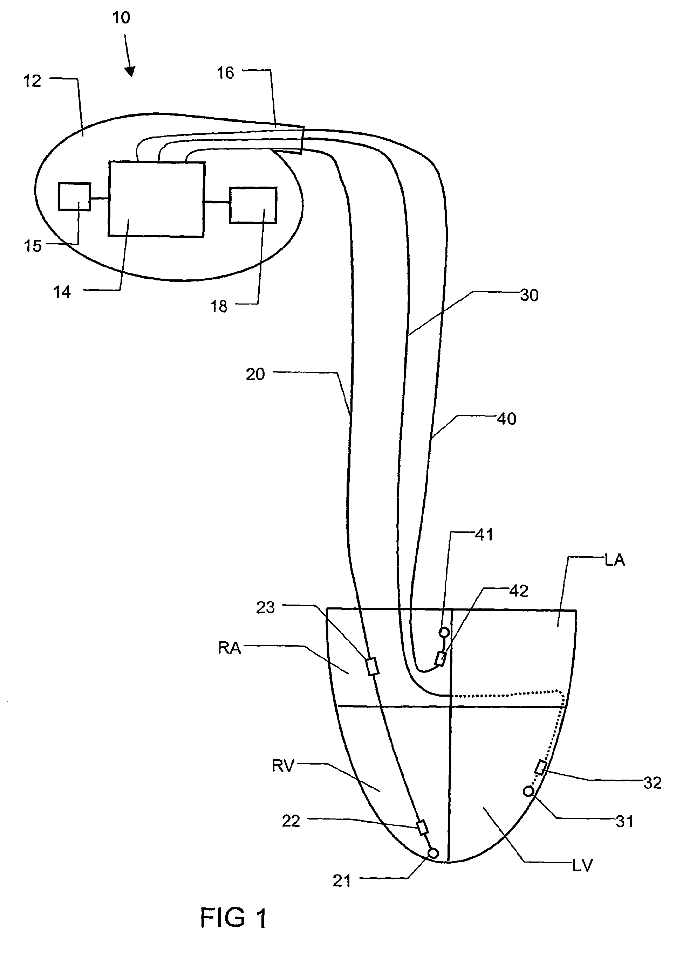

Fig 1 shows schematically an implantable heart monitoring and stimulating system according to the invention. This system includes an implantable heart monitoring and stimulation device 10 according to the invention. The device 10 comprises a housing 12. Inside the housing 12 a control circuit 14 is arranged. The device 10 may constitute a pacemaker which is also able to detect electrical signals from a heart. Since such a device is well known to a person skilled in the art, the details of this device will not be described more closely here. The device comprises a connector portion 16. Different leads 20, 30, 40 may be connected to the control circuit 14 via the connector portion 16. The device comprises a memory 15 connected to the control circuit 14. In said memory 15 data may be stored. Furthermore, the device 10 may include an activity sensor 18 in order to enable the sensing of the activity level of a living being into which the device 10 is implanted.

The device 10 may also be arranged to sense different physiological parameters, such as the blood pressure, the oxygen content in the blood, metabolic substances in the blood or other physiological parameters. Such parameters may be sensed by sensor means connected to the device 10. The sensor means may for example be arranged on one of the leads 20, 30, 40. One such sensor means is indicated by 23.

Fig 1 thus shows three leads 20, 30, 40 connected to the device 10. The number of the leads may be more or less than three. The first lead 20 includes a first sensing member 21, 22. In this case the sensing member 21, 22 includes two electrode surfaces 21 and 22. The electrode member 21 may be called tip electrode and the electrode member 22 may be called ring electrode. The electrode surfaces 21, 22 are thus arranged as bipolar electrodes. However, it is also possible that the device 10 is connected to unipolar electrodes.

In such an arrangement, the housing 12 usually functions as a second electrode. Devices with unipolar and bipolar electrodes are well known to a person skilled in the art and will therefore not be described more closely here.

According to a preferred embodiment, the first sensing member 21, 22 also fulfils the function of a first stimulation member 21, 22. This member 21, 22 may thus be used to deliver stimulation pulses as well as to detect the electrical activity of the heart.

Fig 1 shows schematically a heart with a right atrium RA, a right ventricle RV, a left atrium LA and a left ventricle LV. The first lead 20 is introduced such that the first sensing and stimulation member 21, 22 is positioned in the right ventricle RV.

A second lead 30 includes a second sensing member 31, 32. This member 31, 32 may also function as a second stimulation member 31, 32. The second sensing and stimulation member 31, 32 is arranged such that it can sense and stimulate the left ventricle LV of the heart. The second lead 30 may for example be introduced via the right atrium into the coronary sinus such that the member 31, 32 is positioned in the middle or great cardiac vein. How to introduce the second lead 30 in this manner is known to a person skilled in the art as indicated in some of the above mentioned documents.

Fig 1 also shows a third lead 40 with a sensing and stimulation member 41, 42 positioned in the right atrium RA.

The different leads 20, 30, 40 of course comprise conductors (not shown) in order to conduct signals between the device 10 and the sensing and stimulation members 21, 22, 23, 31, 32, 41, 42. The device 10 together with attached leads 20, 30, 40 thus constitute a system according to the invention.

According to the invention, the control circuit 14 is thus adapted to, via the first sensing member 21, 22, sense cardiac events related to the right ventricle RV. Furthermore, the control circuit is arranged to, via the second sensing member 31, 32, sense cardiac events related to the left ventricle LV. Furthermore, the control circuit 14 is adapted to deliver stimulation pulses to the first 21, 22 and second 31, 32 stimulation members in order to stimulate the right RV and left LV ventricle, respectively. The control circuit 14 is also arranged to enable the following:
a) determine the time T1, T2, T3, within a heart cycle, between a first event related to the right ventricle RV and a second event related to the left ventricle LV, or vice versa,
b) store the determined time T1, T2, T3 in the memory 15,
c) wherein the control circuit 14 is arranged to perform the steps a) and b) at a plurality of occasions and to store the corresponding determined times T1, T2, T3 in said memory 15.

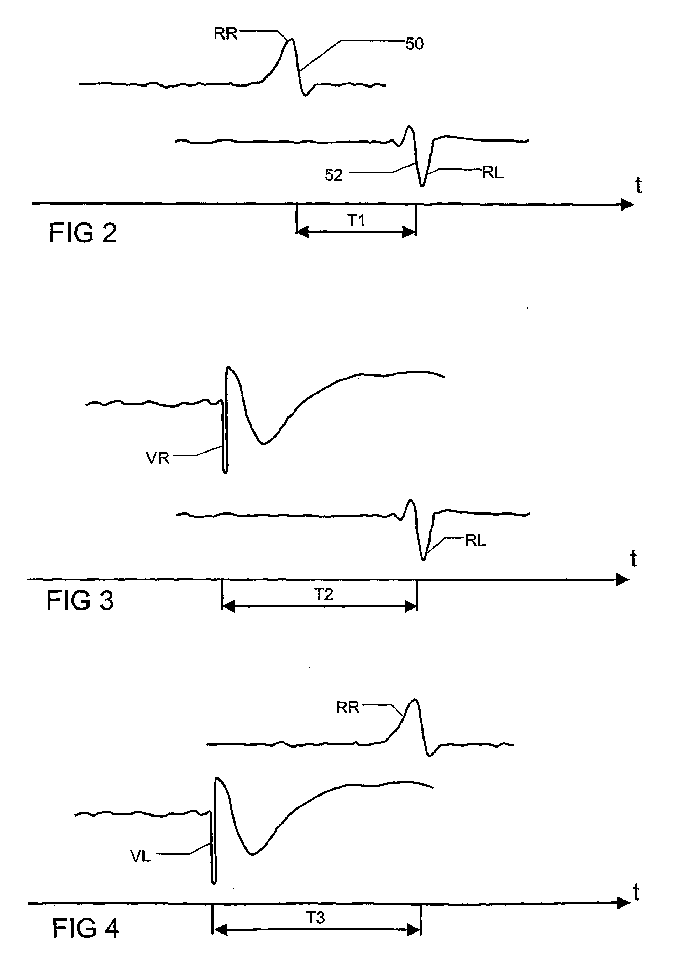

With reference to Fig 2, 3 and 4 it will now be explained what said events may signify. The upper graph in each of these figures relates to the right ventricle and the lower graph relates to the left ventricle. These graphs are shown on a time scale t in order to see when the different events occur in time.

Fig 2 shows a detected R-wave RR of the right ventricle RV. This wave RR may thus be detected with a help of the first sensing member 21, 22. Fig 2 also shows a detected R-wave RL of the left ventricle LV. This wave RL may thus be detected by the second sensing member 31, 32. The time between the waves RR and RL is indicated by T1. In a healthy heart the time T1 is often less than 20ms. The time T1 in a healthy heart depends, inter alia, on where in relation to the ventricles the sensing is done. However, in for example a heart suffering from a bundle branch block, the time T1 is essentially longer. The occurrence of each wave RR and RL may be defined in different manners. It is for example possible to determine the time between the peaks of each wave RR and RL. Another possibility is for example to detect the maximum negative derivative of each wave RR, RL. The point of the maximum negative derivative of the wave RR is indicated by 50. The point of the maximum negative derivative of the wave RL is indicated by 52. According to an embodiment of the invention, the control circuit 14 is thus arranged to determine the time T1 and to store this time T1 in the memory 15. The control circuit 14 is arranged to carry out different determinations of T1 at different occasions and to store the different determined values of T1 in the memory 15. it is thereby possible to see how T1 has changed over time. During the heart cycles when T1 is determined, the control circuit 14 is arranged such that no stimulation pulses are delivered to the first 21, 22 and second 31, 32 stimulation members.

The upper graph of Fig 3 shows a stimulation pulse VR delivered to the right ventricle RV with the help of the first stimulation member 21, 22. The lower graph in Fig 3 shows a detected R-wave RL of the left ventricle LV. This wave RL may thus be detected by the second sensing member 31, 32. The control circuit 14 is arranged to determine the time T2 between the stimulation pulse VR and the R-wave RL. Also in this case the occurrence of the R-wave RL may be defined in different manners, for example as the maximum positive or negative derivative. Since the stimulation pulse VR is delivered by the control circuit 14, the delivery of this pulse VR may trigger a time counter to start measuring the time T2. The occurrence of the R-wave RL then determines the end of the time interval T2. The control circuit 14 is arranged such that during the heart cycles when the time T2 is determined, no stimulation pulse is delivered to the left ventricle LV, i.e. no stimulation pulse is delivered to the second stimulation member 31, 32. The control circuit 14 is arranged to determine the time T2 at different occasions and to store the determined values of T2 in the memory 15.

The lower graph in Fig 4 shows a stimulation pulse VL delivered to the left ventricle LV with the help of the second stimulation member 31, 32. The upper graph in this figure shows a detected R-wave RR originating from the right ventricle RV. This wave RR may thus be detected by the first sensing member 21, 22. The control circuit 14 is arranged to determine the time T3 between the delivered stimulation pulse VL and the sensed R-wave RR. This can be done in an analogous manner to that which has been described above. The control circuit 14 is arranged such that no stimulation pulse is delivered to the right ventricle RV during the heart cycles when the time T3 is determined. The control circuit 14 is arranged such that the time T3 is determined at different occasions and the different values of T3 are stored in the memory 15.

According to the invention, at each occasion one of the values T1, T2, T3 or any two of the values T1, T2, T3 are determined. According to one embodiment of the invention, the control circuit 14 is arranged such that at each occasion all three times T1, T2, T3 in accordance with the above are determined. It may be noted that the mentioned "occasion" thus may comprise a short time span within which the different values T1, T2, T3 are being determined. It may also be noted that it is possible that at each "occasion" several measures of the time in question (for example T1) are done and that an average value of these measures is stored as the determined time T1. In case two or more of the values T1, T2 and T3 are being determined at an occasion, it is possible to store each determined value T1, T2, T3 separately from each other in the memory 15. Alternatively, it is also possible to store a combination (for example an average value) of these values T1, T2 and T3 in the memory 15.

According to a preferred embodiment, the control circuit 14 is arranged such that is receives an indication of the activity level of the human or animal being into which the device 10 is implanted. The control circuit 14 may hereby be arranged to choose said occasions, at which the times T1, T2, T3 are determined, when the activity level is at a predetermined level or with a predetermined range, preferably at a low level of activity. Since all the different occasions are chosen at approximately the same level or activity, a better comparison of the different determined times can be done. The occasions may for example be chosen when the human or animal being in question is asleep.

The control circuit 14 is preferably arranged such that the occasions occur (i.e. the times are determined and stored) at regular intervals. For example, this can be done at least once a month, preferably at least once a week. In order to save space in the memory 15, the occasions should not occur too frequently. For example not more often than each hour, and preferably not more often than each day. The determination of the times may thus for example be done once a day or once a week. It is of course within the scope of the invention that said occasions can occur more often than each hour.

According to one embodiment, the control circuit 14 is arranged to detect at least one pacing parameter or physiological parameter, for example the pacing rate, blood pressure, the oxygen content in the blood or the content of a metabolic substance in the blood or the above mentioned activity level of the human or animal being into which the device is implanted. According to this embodiment, the control circuit 14 is arranged to determine the time T1, T2, T3 at different values of the detected parameter and to store these times and measured values in the memory 15. When analysing the content in the memory 15, it may thereby be seen how the determined times T1, T2, T3 vary in dependence on the mentioned parameter.

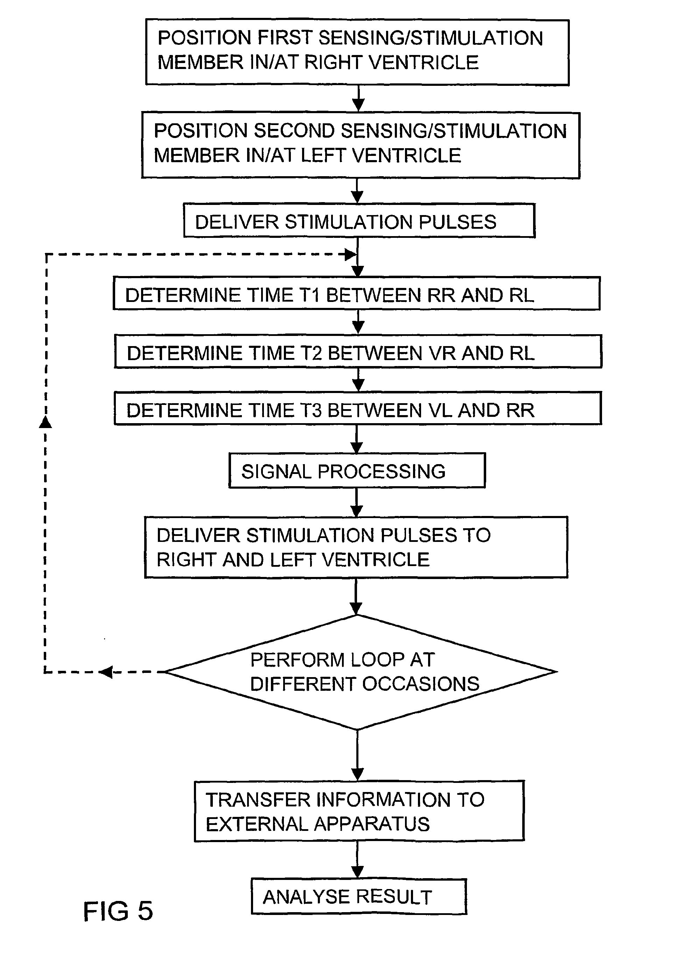

As described above, the invention also concerns an implantable heart monitoring and stimulating system including the device 10 and at least a first 20 and a second 30 lead. The invention also concerns the use of such a system. With reference to Fig 5, a use of this system will now be described. At the same time, the function of the device 10 is clear from Fig 5. According to this use, the device 10 is implanted in a human or animal being and the first sensing/stimulation member 21, 22 is positioned in or at the right ventricle RV. The second sensing/stimulation member 31, 32 is positioned in or at the left ventricle LV. As explained above, this member 31, 32 may for example be positioned in the middle or great cardiac vein. The control circuit 14 is arranged such that it is able to deliver stimulation pulses to the right RV and left LV ventricles. Furthermore, at a certain occasion, the time T1 between RR and RL is determined and stored in the memory 15. As mentioned above, no stimulation pulses are delivered during the heart cycles when T1 is being determined. Moreover, according to a preferred embodiment also the time T2 between VR and RL is determined and stored in the memory 15 in accordance with the above description. Furthermore, the time T3 between VL and RR is determined and stored in the manner described above.

According to one embodiment, the control circuit 14 may be arranged for processing the measured times T1, T2, T3, for example in order to form a combined or average value as explained above and to store this value in the memory 15. When the measurements at one occasion have been carried out, the device 10 is normally set to deliver stimulation pulses to the left LV and right RV ventricles. At a later occasion, the times T1, T2, T3 are determined again in accordance with what has been described above.

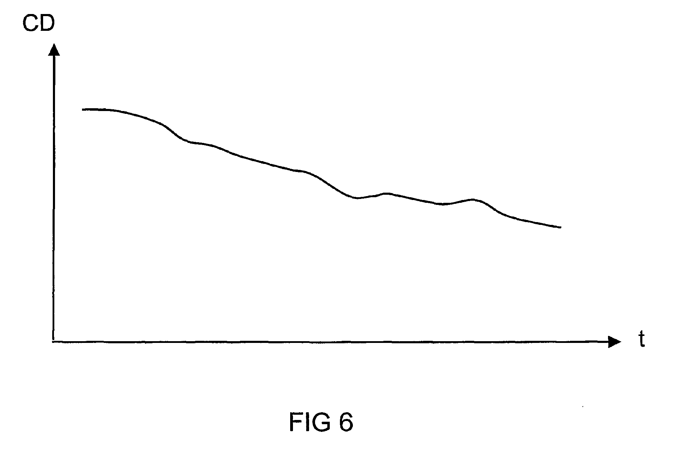

When the loop shown in Fig 5 has been performed a number of times, the information stored in the memory 15 is transferred to an external apparatus, for example to a computer, which does not form part of the implanted system. This transfer may for example be done in connection with a medical check-up, for example once every half year or once a year. The result is analysed in order to see how the heart condition has changed with time. For example, it is possible to form a graph which shows how the determined time varies between the different occasions. Fig 6 shows schematically such a graph. The Y-axis here shows the conduction delay CD between the right RV and left LV ventricles. This conduction delay CD may be represented by either T1, T2 or T3 or by a value which is a combination of these times. The X-axis shows the time. The shown time may for example cover half a year or a year. The graph thus shows whether the conduction between the two ventricles has improved or has gotten worse. In the illustrated example the conduction delay CD has become shorter, which indicates that the heart condition has improved.

The invention is particularly useful in order to monitor the change of the heart condition for a human or animal being, when said being suffers from cardiomyopathy. In particular, when said being suffers from a bundle branch block, primarily a left bundle branch block, the invention is particularly useful. When the being in question suffers from such a block, the electrical signals in the heart are not conducted in the normal manner but are transferred via the heart tissue between the ventricles. As explained above, the conduction delay CD between the ventricles here depends, inter alia, on whether the left ventricle LV has been enlarged or not. By monitoring the determined times T1, T2, T3, an indication of the delay in the conduction of the signals between the ventricles is thus obtained. It is thereby possible to monitor how the heart condition changes with time.

The invention is not limited to the described embodiments but may be varied and modified within the scope of the following claims.

## Claims

1. An implantable heart monitoring and stimulating device (10) comprising:
a control circuit (14),
a memory (15) connected to said control circuit (14),
said control circuit (14) being adapted to be connected to at least a first (21, 22) and a second (31, 32) sensing member, the first sensing member (21, 22) being adapted for sensing cardiac events related to the right ventricle (RV) of the heart and the second sensing member (31, 32) being adapted for sensing cardiac events related to the left ventricle (LV) of the heart, wherein the control circuit (14) is arranged to be able to receive signals from said first (21, 22) and second (31, 32) sensing members such that cardiac events of said right (RV) and left (LV) ventricles are detectable by said control circuit (14),
said control circuit (14) also being adapted to be connected to at least a first (21, 22) and a second (31, 32) stimulation member, the first stimulation member (21, 22) being adapted for electrically stimulating the right ventricle (RV) of the heart and the second stimulation member (31, 32) being adapted for electrically stimulating the left ventricle (LV) of the heart, wherein the control circuit (14) is arranged to be able to transmit stimulation pulses to said first (21, 22) and second (31, 32) stimulation members in order to stimulate said right (RV) and left (LV) ventricles, **characterised in that**
said control circuit (14) is also arranged to at least enable the following:
a) determine the time (T1, T2, T3), within a heart cycle, between a first event related to the right ventricle (RV) and a second event related to the left ventricle (LV), or vice versa,
c) store the determined time (T1, T2, T3) in said memory (15),
c) wherein said control circuit (14) is arranged to perform steps a) and b) at a plurality of occasions and to store the corresponding determined times (T1, T2, T3) in said memory (15).

2. An implantable heart monitoring and stimulating device (10) according to claim 1, wherein said first event is the occurrence of an R-wave (RR) sensed by said first sensing member (21, 22) and said second event is the occurrence of an R-wave (RL) sensed by said second sensing member (31, 32), or vice versa, wherein said control circuit (14) is arranged such that no stimulation pulses are delivered to said first (21, 22) and second (31, 32) stimulation members at least for the heart cycles when said time (T1) between said sensed R-waves (RR, RL) is being determined.

3. An implantable heart monitoring and stimulating device (10) according to claim 1 or 2, wherein said first event is the delivery of a stimulation pulse (VR) to said first stimulation member (21, 22) and said second event is the occurrence of an R-wave (RL) sensed by said second sensing member (31, 32), wherein said control circuit (14) is arranged such that no stimulation pulse is delivered to said second stimulation member (31, 32) at least for the heart cycles when said time (T2) between said stimulation pulse to said first stimulation member (21, 22) and said occurrence of an R-wave (RL) sensed by said second sensing member (31, 32) is being determined.

4. An implantable heart monitoring and stimulating device (10) according to any of the preceding claims, wherein said first event is the delivery of a stimulation pulse (VL) to said second stimulation member (31, 32) and said second event is the occurrence of an R-wave (RR) sensed by said first sensing member (21, 22), wherein said control circuit (14) is arranged such that no stimulation pulse is delivered to said first stimulation member (21, 22) at least for the heart cycles when said time (T3) between said stimulation pulse (VL) to said second stimulation member (31, 32) and said occurrence of an R-wave (RR) sensed by said first sensing member (21, 22) is being determined.

5. An implantable heart monitoring and stimulating device (10) according to claims 2, 3 and 4, wherein said control circuit (14) is arranged such that at each occasion all three times (T1, T2, T3) in accordance with claim 2, 3 and 4 are determined.

6. An implantable heart monitoring and stimulating device (10) according to any of the preceding claims, arranged for sensing the activity level of a human or animal being in which the device (10) is implanted, wherein the control circuit (14) is arranged to choose said occasions to carry out said determination of the time (T1, T2, T3) when the sensed activity level is of a predetermined level or within a predetermined range.

7. An implantable heart monitoring and stimulating device (10) according to claim 6, wherein said predetermined activity level or range implies a low level of activity.

8. An implantable heart monitoring and stimulating device (10) according to any of the preceding claims, wherein said control circuit (14) is arranged such that said occasions occur at least once a month.

9. An implantable heart monitoring and stimulating device (10) according to any of the preceding claims, wherein said control circuit (14) is arranged such that there is at least one hour between said occasions.

10. An implantable heart monitoring and stimulating device (10) according to any of the preceding claims, wherein said control circuit (14) is arranged to detect at least one pacing or physiological parameter, such as the pacing rate, blood pressure, oxygen content in the blood, metabolic substance in the blood or the activity level of the human or animal being in which the device is implanted, wherein the control circuit (14) is arranged to determine said time (T1, T2, T3) at different values of said parameter.

11. An implantable heart monitoring and stimulating system comprising:
a heart monitoring and stimulating device (10) according to any of the preceding claims, and
a first (20) and a second (30) lead connected to said device (10), wherein said first sensing member (21, 22) is arranged on said first lead (20) and said second sensing member (31, 32) is arranged on said second lead (30).

12. An implantable heart monitoring and stimulating system according to claim 11, wherein said first stimulation member (21, 22) is arranged on said first lead (20) and said second stimulation member (31, 32) is arranged on said second lead (30).

13. An implantable heart monitoring and stimulating system according to claim 12, wherein said first stimulation member (21, 22) is the same member as said first sensing member (21, 22) and said second stimulation member (31, 32) is the same member as said second sensing member (31, 32).

## Patentansprüche

1. Implantierbare Herzüberwachungs- und -stimulationsvorrichtung (10) enthaltend:
eine Steuerschaltung (14),
einen Speicher (15), der mit der genannten Steuerschaltung (14) verbunden ist,
wobei die Steuerschaltung (14) ausgelegt ist, mit wenigstens einem ersten (21, 22) und wenigstens einem zweiten (31, 32) Sensorbauteil verbunden zu werden, das erste Sensorbauteil (21, 22) zum Abfühlen von Herzereignissen ausgelegt ist, die sich auf den rechten Ventrikel (RV) des Herzens beziehen und das zweite Sensorbauteil (31, 32) zum Abfühlen von Herzereignissen ausgelegt ist, die sich auf den linken Ventrikel (LV) des Herzens beziehen, ferner die Steuerschaltung (14) so ausgebildet ist, dass sie in der Lage ist, Signale von dem genannten ersten (21, 22) und dem genannten zweiten (31, 32) Sensorbauteil so zu empfangen, dass Herzereignisse des genannten rechten (RV) und des genannten linken (LV) Ventrikels durch die genannte Steuerschaltung (14) detektierbar sind,
die genannte Steuerschaltung (14) auch ausgelegt ist, mit wenigstens einem ersten (21, 22) und wenigstens einem zweiten (31, 32) Stimulationsbauteil verbunden zu werden, wobei das erste Stimulationsbauteil (21, 22) zum elektrischen Stimulieren des rechten Ventrikels (RV) des Herzens ausgelegt ist und das zweite Stimulationsbauteil (31, 32) zum elektrischen Stimulieren des linken Ventrikels (LV) des Herzens ausgelegt ist, ferner die Steuerschaltung (14) so ausgebildet ist, dass sie in der Lage ist, Stimulationsimpulse zu dem genannten ersten (21, 22) und dem genannten zweiten (31, 32) Stimulationsbauteil zu senden, um den genannten rechten (RV) und den genannten linken (LV) Ventrikel zu stimulieren, **dadurch gekennzeichnet, dass**
die genannte Steuerschaltung (14) auch so ausgebildet ist, dass sie wenigstens das Folgende ermöglicht:
a) Ermittle die Zeit (T1, T2, T3), innerhalb eines Herzzyklus, zwischen einem ersten, sich auf den rechten Ventrikel (RV) beziehenden Ereignis und einem zweiten, sich auf den linken Ventrikel (LV) beziehenden Ereignis, oder umgekehrt,
b) Speichere die festgestellte Zeit (T1, T2, T3) in dem genannten Speicher (15),
c) wobei die genannte Steuerschaltung (14) so ausgebildet ist, dass sie die Schritte a) und b) bei einer Mehrzahl von Anlässen durchführt, und die dazugehörigen festgestellten Zeiten (T1, T2, T3) in dem genannten Speicher speichert.

2. Implantierbare Herzüberwachungs- und -stimulationsvorrichtung (10) nach Anspruch 1, wobei das genannte erste Ereignis das Auftreten einer durch das genannte erste Sensorbauteil (21, 22) abgefühlten R-Welle (RR) und das genannte zweite Ereignis das Auftreten einer durch das genannte zweite Sensorbauteil (31, 32) abgefühlten R-Welle (RL) ist, oder umgekehrt, wobei die genannte Steuerschaltung (14) so ausgebildet ist, dass zu dem genannten ersten (21, 22) und dem genannten zweiten (31, 32) Stimulationsbauteil wenigstens für die Herzzyklen, wenn die genannte Zeit (T1) zwischen den abgefühlten R-Wellen (RR, RL) ermittelt wird, keine Stimulationsimpulse geliefert werden.

3. Implantierbare Herzüberwachungs- und -stimulationsvorrichtung (10) nach Anspruch 1 oder 2, wobei das genannte erste Ereignis die Ausgabe eines Stimulationsimpulses (VR) zu dem genannten ersten Stimulationsbauteil (21, 22) und das genannte zweite Ereignis das Auftreten einer durch das genannte zweite Sensorbauteil (31, 32) abgefühlten R-Welle (RL) ist, wobei die genannte Steuerschaltung (14) so ausgebildet ist, dass zu dem genannten zweiten Stimulationsbauteil (31, 32) kein Stimulationsimpuls wenigstens für die Herzzyklen ausgegeben wird, wenn die genannte Zeit (T2) zwischen dem genannten Stimulationsimpuls zu dem genannten ersten Stimulationsbauteil (21, 22) und dem genannte Auftreten einer durch das genannte zweite Sensorbauteil (31, 32) abgefühlte R-Welle (RL) ermittelt wird.

4. Implantierbare Herzüberwachungs- und -stimulationsvorrichtung (10) nach einem der vorhergehenden Ansprüche, wobei das genannte erste Ereignis die Ausgabe eines Stimulationsimpulses (VL) zu dem genannten zweiten Stimulationsbauteil (31, 32) und das genannte zweite Ereignis das Auftreten einer durch das genannte erste Sensorbauteil (21, 22) abgefühlte R-Welle (RR) ist, wobei die genannte Steuerschaltung (14) so ausgebildet ist, dass zu dem genannte ersten Stimulationsbauteil (21, 22) kein Stimulationsimpuls wenigstens für die Herzzyklen ausgegeben wird, wenn die genannte Zeit (T3) zwischen dem genannten Stimulationsimpuls (VL) zu dem genannten zweiten Stimulationsbauteil (31, 32) und dem genannten Auftreten einer durch das genannte erste Sensorbauteil (21, 22) abgefühlten R-Welle (RR) ermittelt wird.

5. Implantierbare Herzüberwachungs- und -stimulationsvorrichtung (10) gemäß den Ansprüchen 2, 3 bzw. 4, wobei die genannte Steuerschaltung (14) so ausgebildet ist, dass bei jedem Anlass alle drei Zeiten (T1, T2, T3) gemäß den Ansprüchen 2, 3 und 4 ermittelt werden.

6. Implantierbare Herzüberwachungs- und -stimulationsvorrichtung (10) nach einem der vorhergehenden Ansprüche, angeordnet zum Abfühlen des Aktivitätspegels eines menschlichen oder tierischen Wesens, in das die Vorrichtung (10) implantiert ist, wobei die Steuerschaltung (14) ausgebildet ist, die genannten Anlässe auszuwählen, um die genannte Ermittlung der Zeit (T1, T2, T3) auszuführen, wenn der abgefühlte Aktivitätspegel ein vorbestimmter Pegel ist oder innerhalb eines vorbestimmten Bereiches liegt.

7. Implantierbare Herzüberwachungs- und -stimulationsvorrichtung (10) nach Anspruch 6, wobei der genannte vorbestimmte Aktivitätspegel oder Bereich einen niedrigen Aktivitätspegel bedeutet.

8. Implantierbare Herzüberwachungs- und -stimulationsvorrichtung (10) nach einem der vorhergehenden Ansprüche, wobei die genannte Steuerschaltung (14) so ausgebildet ist, dass die genannten Anlässe wenigstens einmal pro Monat auftreten.

9. Implantierbare Herzüberwachungs- und -stimulationsvorrichtung (10) nach einem der vorhergehenden Ansprüche, wobei die genannte Steuerschaltung (14) so ausgebildet ist, dass wenigstens eine Stunde zwischen den genannten Anlässen liegt.

10. Implantierbare Herzüberwachungs- und -stimulationsvorrichtung (10) nach einem der vorhergehenden Ansprüche, wobei die genannte Steuerschaltung (14) so ausgebildet ist, dass sie wenigstens einen Stimulationsparameter oder physiologischen Parameter, wie die Stimulationsfrequenz, den Blutdruck, den Sauerstoffgehalt im Blut, die metabolische Substanz im Blut oder den Aktivitätspegel des menschlichen oder tierischen Wesens detektiert, in das die Vorrichtung implantiert ist, wobei die Steuerschaltung (14) so ausgebildet ist, dass sie die genannte Zeit (T1, T2, T3) bei verschiedenen Werten des genannten Parameters ermittelt.

11. Implantierbares Herzüberwachungs- und -stimulationssystem, enthaltend:
eine Herzüberwachungs- und -stimulationsvorrichtung (10) nach einem der vorhergehenden Ansprüche, und
eine erste (20) und eine zweite (30) Leitung, die mit der genannten Vorrichtung (10) verbunden sind, wobei das genannte erste Sensorbauteil (21, 22) an der genannten ersten Leitung (20) angeordnet ist und das genannte zweite Sensorbauteil (31, 32) an der genannten zweiten Leitung (30) angeordnet ist.

12. Implantierbares Herzüberwachungs- und -stimulationssystem nach Anspruch 11, wobei das genannte erste Stimulationsbauteil (21, 22) an der genannten ersten Leitung (20) angeordnet ist und das genannte zweite Stimulationsbauteil (31, 32) an der genannten zweiten Leitung (30) angeordnet ist.

13. Implantierbares Herzüberwachungs- und -stimulationssystem nach Anspruch 12, wobei das genannte erste Stimulationsbauteil (21, 22) das selbe Bauteil wie das genannte erste Sensorbauteil (21, 22) ist und das genannte zweite Stimulationsbauteil (31, 32) das selbe Bauteil wie das genannte zweite Sensorbauteil (31, 32) ist.

## Revendications

1. Dispositif (10) implantable de surveillance et de stimulation cardiaques, comprenant :
un circuit (14) de commande,
une mémoire (15) reliée au circuit (14) de commande,
le circuit (14) de commande étant conçu pour être connecté au moins à des premier (21, 22) et deuxième (31, 32) éléments de détection, le premier élément (21, 22) de détection étant conçu pour détecter des événements cardiaques associés au ventricule droit (VD) du coeur et le deuxième élément (31, 32) de détection étant conçu pour détecter des événements cardiaques associés au ventricule gauche (VG) du coeur, le circuit (14) de commande étant agencé pour être apte à recevoir des signaux en provenance des premier (21, 22) et deuxième (31, 32) éléments de détection, de manière à ce que des événements cardiaques des ventricules droit (VD) et gauche (VG) soient détectables par le circuit (14) de commande,
le circuit (14) de commande étant également conçu pour être connecté au moins à des premier (21, 22) et deuxième (31, 32) éléments de stimulation, le premier élément (21, 22) de stimulation étant conçu pour stimuler électriquement le ventricule droit (VD) du coeur et le deuxième élément (31, 32) de stimulation étant conçu pour stimuler électriquement le ventricule gauche (VG) du coeur, le circuit (14) de commande étant agencé pour être apte à émettre des impulsions de stimulation vers les premier (21, 22) et deuxième (31, 32) éléments de stimulation, afin de stimuler les ventricules droit (VD) et gauche (VG),
**caractérisé en ce que** :
le circuit (14) de commande est également agencé pour permettre au moins ce qui suit :
a) déterminer le temps (T1, T2, T3) à l'intérieur d'un cycle cardiaque, entre un premier événement associé au ventricule droit (VD) et un deuxième événement associé au ventricule gauche (VD) ou vice versa,
b) stocker le temps déterminé (T1, T2, T3) dans la mémoire (15),
c) dans lequel le circuit (14) de commande est agencé pour exécuter les étapes a) et b) en une pluralité d'occasions et pour stocker les temps (T1, T2, T3) déterminés correspondants dans la mémoire (15).

2. Dispositif (10) implantable de surveillance et de stimulation cardiaques suivant la revendication 1, dans lequel le premier événement est l'apparition d'une onde R (RD) détectée par le premier élément (21, 22) de détection et le deuxième événement est l'apparition d'une onde R (RG) détectée par le deuxième élément (31, 32) de détection ou vice versa, dans lequel le circuit (14) de commande est agencé de telle sorte qu'aucune impulsion de stimulation n'est délivrée aux premier (21, 22) et deuxième (31, 32) éléments de stimulation au moins pendant les cycles cardiaques où le temps (T1) entre les ondes R (RD, RG) détectées est déterminé.

3. Dispositif (10) implantable de surveillance et de stimulation cardiaques suivant la revendication 1 ou 2, dans lequel le premier événement est l'envoi d'une impulsion (VD) de stimulation au premier élément (21, 22) de stimulation et le deuxième événement est l'apparition d'une onde R (RG) détectée par le deuxième (31, 32) élément de détection, dans lequel le circuit (14) de commande est agencé de telle sorte qu'aucune impulsion de stimulation n'est envoyée au deuxième (31, 32) élément de stimulation au moins pendant les cycles cardiaques où le temps (T2) entre l'impulsion de stimulation émise vers le premier élément (21, 22) de stimulation et l'apparition d'une onde R (RG) détectée par le deuxième élément (31, 32) de détection est déterminé.

4. Dispositif (10) implantable de surveillance et de stimulation cardiaques suivant l'une quelconque des revendications précédentes, dans lequel le premier événement est l'envoi d'une impulsion (VG) de stimulation au deuxième élément (31, 32) de stimulation et le deuxième événement est l'apparition d'une onde R (RD) détectée par le premier élément (21, 22) de détection, dans lequel le circuit (14) de commande est agencé de telle sorte qu'aucune impulsion de stimulation n'est envoyée au premier élément (21, 22) de stimulation au moins pendant les cycles cardiaques où le temps (T3) entre l'impulsion (VG) de stimulation émise vers le deuxième élément (31, 32) de stimulation et l'apparition d'une onde R (RD) détectée par le premier élément (21, 22) de détection est déterminé.

5. Dispositif (10) implantable de surveillance et de stimulation cardiaques suivant les revendications 2, 3 et 4, dans lequel le circuit (14) de commande est agencé de telle sorte qu'en chaque occasion, les trois temps (T1, T2, T3) suivant les revendications 2, 3 et 4 sont déterminés.

6. Dispositif (10) implantable de surveillance et de stimulation cardiaques suivant l'une quelconque des revendications précédentes, conçu pour détecter le niveau d'activité d'un humain ou d'un animal dans le corps duquel le dispositif (10) est implanté, dans lequel le circuit (14) de commande est conçu pour choisir les occasions où effectuer la détermination du temps (T1, T2, T3) lorsque le niveau d'activité détecté est un niveau déterminé à l'avance ou se situe à l'intérieur d'une plage déterminée à l'avance.

7. Dispositif (10) implantable de surveillance et de stimulation cardiaques suivant la revendication 6, dans lequel le niveau ou la plage d'activité déterminé(e) à l'avance implique un niveau faible d'activité.

8. Dispositif (10) implantable de surveillance et de stimulation cardiaques suivant l'une quelconque des revendications précédentes, dans lequel le circuit (14) de commande est agencé de telle sorte que les occasions se produisent au moins une fois par mois.

9. Dispositif (10) implantable de surveillance et de stimulation cardiaques suivant l'une quelconque des revendications précédentes, dans lequel le circuit (14) de commande est agencé de telle sorte qu'il s'écoule au moins une heure entre les occasions.

10. Dispositif (10) implantable de surveillance et de stimulation cardiaques suivant l'une quelconque des revendications précédentes, dans lequel le circuit (14) de commande est conçu pour détecter au moins un paramètre de stimulation ou un paramètre physiologique, tel que la fréquence de stimulation, la pression artérielle, la teneur en oxygène du sang, une substance métabolique dans le sang ou le niveau d'activité de l'humain ou de l'animal dans le corps duquel le dispositif est implanté, dans lequel le circuit (14) de commande est conçu pour déterminer le temps (T1, T2, T3) à différentes valeurs de ce paramètre.

11. Système implantable de surveillance et de stimulation cardiaques, comprenant :
un dispositif (10) de surveillance et de stimulation cardiaques, suivant l'une quelconque des revendications précédentes, et
des première (20) et deuxième (30) dérivations reliées au dispositif (10), le premier élément (21, 22) de détection étant placé sur la première dérivation (20) et le deuxième élément (31, 32) de détection étant placé sur la deuxième dérivation (30).

12. Système implantable de surveillance et de stimulation cardiaques suivant la revendication 11, dans lequel le premier élément (21, 22) de stimulation est placé sur la première dérivation (20) et le deuxième élément (31, 32) de stimulation est placé sur la deuxième dérivation (30).

13. Système implantable de surveillance et de stimulation cardiaques suivant la revendication 12, dans lequel le premier élément (21, 22) de stimulation est le même élément que le premier élément (21, 22) de détection et le deuxième élément (31, 32) de stimulation est le même élément que le deuxième élément (31, 32) de détection.
